# EUROPEAN PATENT SPECIFICATION

(11) **EP 4 082 473 B1**
(45) Date of publication and mention of the grant of the patent: **04.11.2026**
(21) Application number: 21211084.5
(22) Date of filing: 27.09.2017
(51) Int. Cl.: A61B 90/00

(54) **BIOPSY SITE MARKER**
BIOPSIESTELLENMARKER
MARQUEUR DE SITE DE BIOPSIE

(30) Priority: 23.11.2016 US 201662425953 P
(43) Date of publication of application: 02.11.2022
(60) Divisional application: 26202652.9
(62) Document number(s) of the earlier application(s) in accordance with Art. 76 EPC: 17784120.2 / 3 544 540
(73) Proprietor: Hologic, Inc., Marlborough, MA 01752 (US)
(72) Inventor: ALVAREZ, Estefania, Marlborough (US); KOZAK, Adam, Marlborough (US); ROBERTSON, Jaimee, Marlborough (US); SPIEGELBERG, Stephen, Marlborough (US)
(74) Representative: Abel & Imray LLP

(56) References cited:
- EP-A1- 3 912 569
- EP-A2- 2 662 041
- WO-A1-2017/207777
- US-A1- 2015 305 750

## Description

### FIELD OF INVENTION

The disclosed inventions relate generally to site markers for breast biopsy procedures. More specifically, the disclosed inventions relate to site markers that are visible under several different imaging modalities.

### BACKGROUND

In the diagnosis and treatment of breast cancer, it is often necessary to perform a biopsy to remove tissue samples from a suspicious mass. The suspicious mass is typically discovered during a preliminary examination involving visual examination, palpation, x-ray, magnetic resonance imaging (MRI), ultrasound imaging or other detection means.

When a suspicious mass is detected, a sample is taken by biopsy, and then tested to determine whether the mass is malignant or benign. This biopsy procedure can be performed by an open surgical technique, or through the use of a specialized biopsy instrument. To minimize surgical intrusion, a small specialized instrument such as a biopsy needle is inserted in the breast while the position of the needle is monitored using an imaging technique such as fluoroscopy, ultrasonic imaging, x-rays, MRI or other suitable imaging techniques.

In one biopsy procedure, referred to as stereotactic needle biopsy, the patient lies on a special biopsy table with her breast compressed between the plates of a mammography apparatus and two separate X-rays are taken from two different points of reference. A computer then calculates the exact position of the mass or lesion within the breast. The coordinates of the lesion are then programmed into a mechanical stereotactic apparatus which advances the biopsy needle into the lesion with precision. At least five biopsy samples are usually taken from locations around the lesion and one from the center of the lesion.

Regardless of the method or instrument used to perform the biopsy, subsequent examination of the surgical site may be necessary, either in a follow up examination or for treatment of a cancerous lesion. Treatment often includes a mastectomy, lumpectomy, radiation therapy, or chemotherapy procedure that requires the surgeon or radiologist to direct surgical or radiation treatment to the precise location of the lesion. Because this treatment might extend over days or weeks after the biopsy procedure, by which time the original features of the tissue may have been removed or altered by the biopsy, it is desirable to insert a site marker into the surgical cavity to serve as a landmark for future identification of the location of the lesion.

Implantable marking devices for marking tissue sites are known in general. WO2017/207777 relates to a marking device for implantation into a tissue, having an elastic compressible and self-expanding support structure encompassing, in an expanded state, an interior space. Further relevant prior art is disclosed in WO 2017/207777 A1.

Known biopsy site markers have been found to have disadvantages in that the site markers are not visible under all available imaging modalities. Moreover, because of this problem, when cancer is found at a biopsy site that has been previously marked with a site marker, due to the poor visibility of the biopsy site marker under ultrasound or other visualization modalities, or lack of differentiation between marker and anatomical features, the patient must undergo an additional procedure that places an additional device within the biopsy site to enable the surgeon to find the biopsy site during surgery. Limited visibility (or none at all when using ultrasound) limits the ability to monitor tumor progression or shrinkage during neo adjuvant chemotherapy with the marker serving as a reference point. One known technique has been to place a breast lesion localization wire at the biopsy site. The localization wire is typically placed at the biopsy site via mammography and/or ultrasound.

Another issue that arises with site markers is migration. When the site markers are typically deployed to the biopsy site, the breast is still under compression. However, when the breast is released from compression, the site marker may migrate within the site or even out of the site through a needle tract created by the biopsy device, thereby preventing a surgeon or radiologist from easily locating the precise location of the lesion or biopsied area.

Accordingly, there is a need for site markers made from biocompatible materials that are visible under various modes of imaging to reduce the number of procedures that patients must undergo in detection and treatment of cancer. There is also a need to limit migration of a site marker when the site marker is placed in a biopsy site.

### SUMMARY

The invention is defined in appended claim 1.

### BRIEF DESCRIPTION OF THE DRAWINGS

The drawings illustrate the design and utility of the disclosed embodiments, in which similar elements are referred to by common reference numerals. In order to better appreciate how the above-recited and other advantages and objects of the disclosed embodiments are obtained, a more particular description is set forth by reference to the accompanying drawings. Understanding that these drawings depict exemplary embodiments of the disclosed inventions, and are not therefore to be considered limiting, the illustrated embodiments will be described and explained with additional specificity and detail through the use of the accompanying drawings in which:
**Fig. 1** is a perspective view of a biopsy site in a human breast showing the breast in section and one or more site markers being implanted in the biopsy cavity;
**Figs. 4A and 4B** are perspective views of a site marker in an expanded state and a contracted state, respectively, according to a third embodiment;
**Figs. 7A and 7B** are plan views of a delivery device prior to marker deployment and after marker deployment, respectively; and
**Fig. 8** is an alternate embodiment of a delivery device for the site markers.

### DETAILED DESCRIPTION OF THE ILLUSTRATED EMBODIMENTS

Biopsy markers are described herein for use in breast biopsy, but could be used in other areas. The markers described herein present a significant performance change from existing technologies without substantially adding to the cost of this fundamentally high volume product. Existing products provide an unacceptably short duration of ultrasound contrast and have been prone to migration from the original biopsy location due to a lack of tissue adhesion to cavity walls, mechanical attachment, or rapid expansion upon deployment. These deficiencies suggest a new device comprised of a hydrogel, or similar technology, surrounding a radiopaque core or a hydrogel contained within a flexible envelope.

Preferably, a biopsy site marker provides permanent radiographic contrast and distinct ultrasound contrast for a minimum of one year (fifty-two weeks) within limited posterior acoustic shadowing. The portion of the marker providing ultrasound contrast may be either permanent or biodegradable. The marker preferably has a "man-made" shape to clearly differentiate it from other biological structure and abnormal features. The permanent portion of the marker preferably sits in the middle of the cavity. The marker is preferably configured to minimize migration and improve tissue adhesion relative to existing cavity structure. The marker preferably swells to fill the biopsy cavity, yet not swell so much that it is palpable by the patient. Delivery of the marker is preferably single-handed and as simple as possible. The shelf life is preferably at least two years.

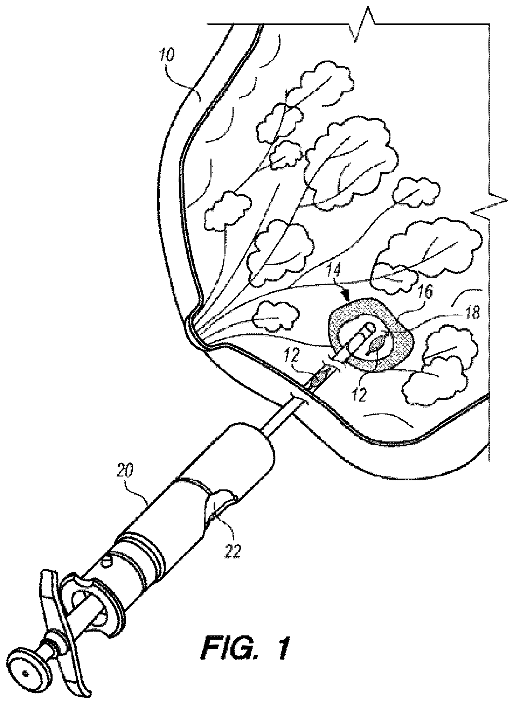

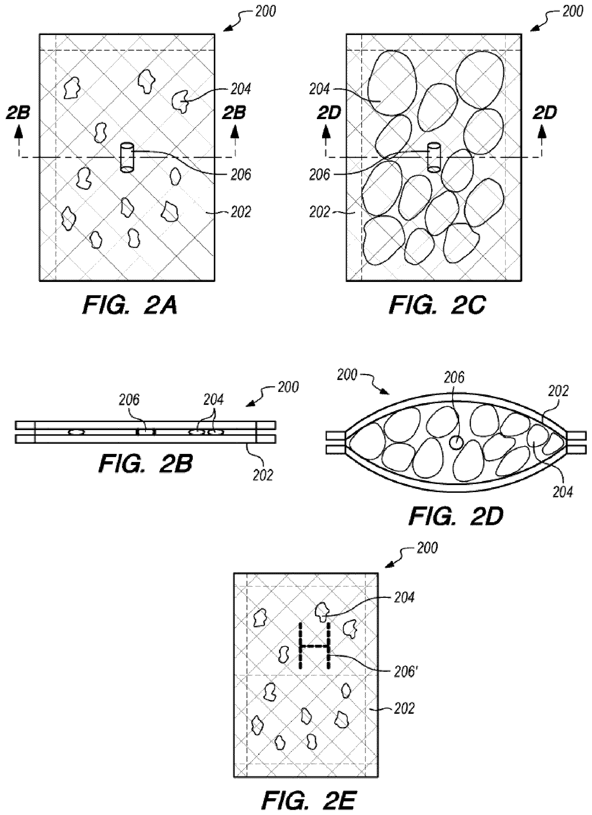

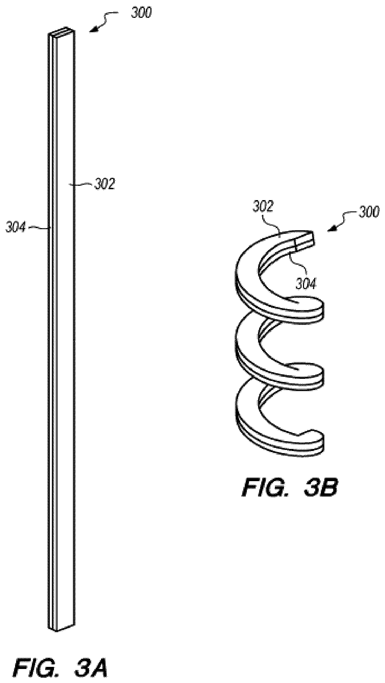

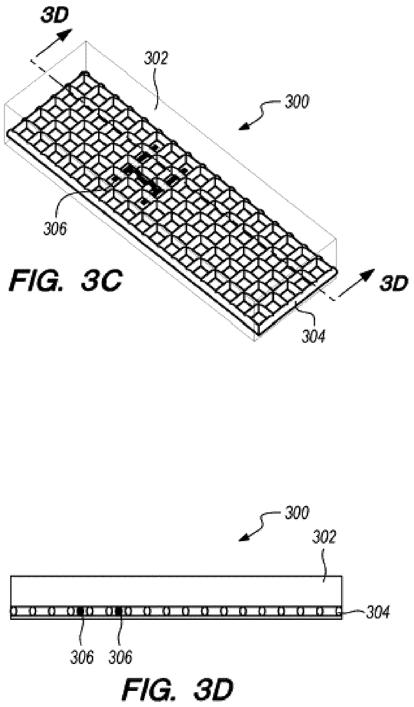

FIG. 1 illustrates a perspective view of a human breast 10 being implanted with a site marker 12 according an embodiment of the disclosure. At a biopsy site 14 is a lesion 16 from which a tissue sample has been removed, resulting in a biopsy cavity 18. One or more site markers 12 are implanted in the biopsy cavity 18 using a marker delivery system 20. In one embodiment, the marker delivery system 20 advanced by sliding it through an inner lumen 22 of a biopsy device (not shown), which avoids the need to withdraw the biopsy device and thereafter insert the marker delivery system 20. Delivering the site marker 12 in the biopsy cavity 18 without withdrawing the biopsy device reduces the amount of tissue damage and enables more accurate placement of the site marker 12. The marker delivery system 20 illustrated in FIG. 1 is exemplary only and it is understood that the site marker embodiments disclosed herein are suitable for use with other marker delivery systems.

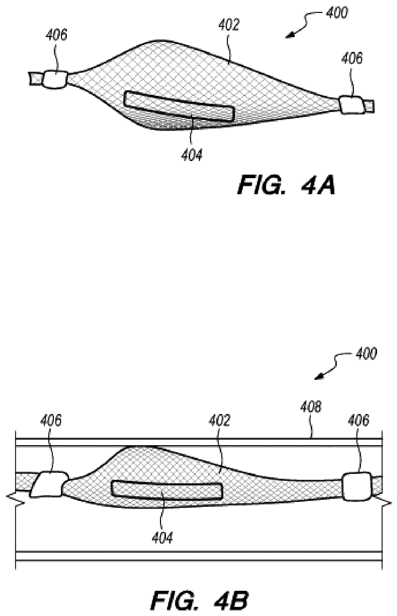

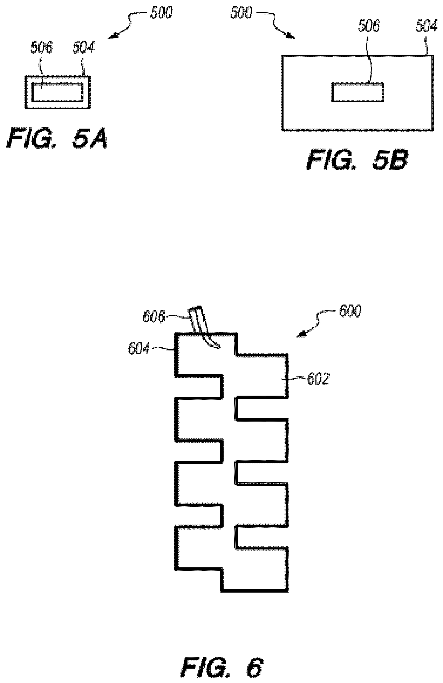

FIGS. 4A-4B illustrate exemplary site marker embodiments according to the present disclosure. In general, the site markers described herein are made from biocompatible materials that have appropriate densities for radiographic imaging, appropriate surface characteristics, and acoustic impedance differences for ultrasonic imaging, and appropriate magnetic characteristics for magnetic resonance imaging. The constituent materials of the site markers are either not biodegradable or can be tuned to degrade over a long time scale. The fifty-two week scale encompasses a typical timespan in which a physician would need visibility of the marker to be able to monitor a full round of neo adjuvant chemo therapy, in order to reduce the tumor significantly leading to lumpectomy. In particular, uniformity of acoustic impedance in the marker is highly desirable. A strong impedance mismatch will result in a clear outline using ultrasound, and the ability of the hydrogels described herein to maintain a constant impedance throughout yields a "clean" and speckle-free image, whereas a hydrogel with heterogenous impedance would yield a speckled image, and may be mistaken for tissue, which is further discussed below.

The site markers disclosed herein in the pre-deployment state are solid compacted structures, in dehydrated states reduced in size from their desired final state. These markers also have a distinct 3D configuration in a hydrated state. Upon deployment in a biopsy cavity, the markers are configured to expand in situ immediately and reach full size within thirty to sixty minutes. The markers may expand axially (in height) and longitudinally (in width) to a size that is larger than the initial size of the biopsy tract. Preferably, the fully expanded marker occupies more than 50% of the diameter of the biopsy cavity. This expansion can be tailored based on the type of polymer used for the superabsorbent hydrogel, the conditions under which the polymer has been prepared (e.g., different processing conditions which may influence e.g. crosslink density and affect swell ratio/swell kinetics), the surface area of the polymer, swelling rate of the polymer, the give or elasticity of a polymer/metal envelope that contains the hydrogel polymer, the processing conditions to set the final/expanded shape and its final dimensions, the amount of saline used for deployment, and the amount of bodily fluids within the biopsy cavity. This tailored expansion could aid in precise marking of the lesion and precise deployment, and preventing the marker from being displaced within the biopsy cavity or biopsy track. The time frame of sixty minutes allows a radiologist to confirm placement after deployment using any imaging modality, and to confirm placement with a mammogram shortly after the marker is deployed. In particular, the markers will start to become visible under U/S and MRI within minutes, and will reach their "final" deployed state within an hour of being deployed in the patient's body This is routine practice as radiologists image at deployment and confirm with a mammogram before a patient leaves the facility. A fully expanded marker preferably has a non-palpable feel at deployment, and in the long term due to the selection of biocompatible materials should trigger a minor tissue inflammatory response with minimal scar formation.

According to embodiments of the invention as shown in Figs. 4A and 4B, a shape memory-based biopsy marker 400 comprises a cage 402 surrounding a superabsorbent hydrogel core 404. The cage 402 has teardrop-like shape, and comprises a superelastic shape memory material. For example, the cage 402 may be made of Nitinol. The marker 400 further includes crimp beads 406 at either end of the cage 402. The crimp beads 406 may be metallic, or alternatively made of a polymer, and are used to close off the respective ends of the Nitinol cage 402, thereby enclosing the hydrogel component 404 within the cage 402 to provide unique X-ray identification. According to the invention welding or knotting may be employed to close off the ends of the cage 402, knotting may for example be realized by a drawstring and cinch mechanism. After the hydrogel core 404 is positioned within the cage 402, the ends of the cage 402 may be closed off with the crimp beads 406. In some embodiments, the crimp beads 406 are metallic, and may comprise titanium, gold, silver, or the like. In one example, the Nitinol cage 402 may be radially compressed and threaded through a 0.8 mm silver plated brass crimp bead 406. In such embodiments, the metallic composition of the crimp bead 406 may provide further radiographic contrast with minimal to no MRI artifacts. Alternatively, the cage 402 may be constructed using Nitinol having a shape transition temperature approximately the same as body temperature, and flushing with warm/hot saline to trigger a thermal transition, i.e. thereby relying on the true shape memory mechanism, rather than rely on superelasticity.

Nitinol is the preferable shape memory material for the cage 402 due to its robust shape memory properties and the ability to tune the activating temperature through composition. In one example, the cage-like structure 402 is made of braided Nitinol tubing. The braiding technique may impart desirable properties in the 3D tubing construct. Specifically, the Nitinol tubing is highly compressible through radial compression and highly expandable through axial compression. After release of either the radial or axial compressive force, the Nitinol tubing elastically contracts to its native state. The extreme diameter changes result from the braided geometry. Properties such as the maximum outer diameter, the braid density, and the elastic response of the tubing can be adjusted through the braiding parameters.

The superelasticity of the Nitinol may be utilized as a means for compact delivery in a small diameter cannula and rapid space filling upon deployment in the biopsy cavity. The Nitinol cage 402 may be set to a permanent expanded shape with a desired tapering diameter and a desired length. For example, the diameter of the cage 402 may be approximately 0.5 cm at the maximum point, and the length of the cage 402 may be approximately 1.0 cm. The shape memory marker 400 may be compressed to a smaller diameter such that the Nitinol tubing forming the cage 402 are in contact with the hydrogel core 404. In this form, the shape memory marker 400 is easily inserted into a cannula 408 for deployment, as shown in Fig. 4B. The size of the cannula 408 is therefore likely limited by the diameter of the hydrogel component 404, although the profile of the crimp beads 406 or alternative cage sealing material, as well as the diameter of the Nitinol tubing/wire forming the braid may also limit cannula size. The shape memory marker 400 may be extruded into the biopsy cavity using a simple push rod, and may be back flushed with saline to accelerate swelling of the hydrogel 404. Upon exiting the cannula 408, immediate elastic expansion assists in precise marking of lesion, localizing the marker 400 and preventing migration from the biopsy site. In addition, the torpedo-like shape may help prevent migration of the marker 400 along the biopsy tract during retraction of the cannula 408. As a larger diameter in the distal portion of the marker, prevents marker migration in the direction of least resistance such as the biopsy tract.

The hydrogel core 404 in the shape memory marker 400 provides ultrasound and MRI contrast. The hydrogel core 404 may, for example, be approximately 0.5 cm long and 0.8 mm in diameter in the dehydrated state. In one embodiment, the hydrogel 404 is a poly(vinyl pyrrolidone-co-ethylene glycol dimethacrylate) (PVPEGDA) gel prepared with the following formulation: 7.5% vinyl pyrrolidone, 2.5% poly(ethylene glycol) dimethyacrylate (700 g/mol), and 1% sodium chloride in water. Relative to the monomer content, 1% 2,2-dimethoxy-2-phenylactophenone may be added as a photoinitiator to enable crosslinking via ultraviolet radiation. The resulting formulation may be cast in quartz tubing (e.g., 2.3 mm nominal ID) to obtain cylindrical hydrogel rods. Lower overall monomer content was used for the hydrogel component 404 of the shape memory marker 400 compared to the hydrogels used in the other marker embodiments. The lower monomer content allows for a higher swell ratio, but reduces the mechanical strength of the gel in the swollen state. The shape memory marker design encapsulates the hydrogel 404 within a cage structure 402, and thus, does not rely on mechanical strength of the hydrogel 404 during potential marker removal.

The shape memory marker 400 is distinct from the other marker embodiments in that no radiopaque element was added solely for the purpose of imaging contrast. In some embodiments, the Nitinol cage 402 and the crimp beads 406 are metallic and inherently radiopaque. Adjustment of the permanent shape of the Nitinol cage 402 or the size, shape, or number of the crimp beads 406 may enable differentiation between multiple biopsy sites.

The shape memory marker 400 (including the crimp beads 406) may be coated with a thin hydrogel layer (e.g., polyethylene glycol (PEG) or the like) or other low friction materials (e.g. ePTFE, silicone, poly(glycerol sebacate) PGS) that may aid in reducing friction of the shape memory marker 400 upon deployment. Such a coating may facilitate deployment of the shape memory marker 400 out of the distal end of a delivery device, and may also prevent shredding of the cage 402 due to friction between the outer surface of the cage 402 and the inner surface of a delivery cannula. This coating may also provide another enhancement for ultrasound visualization. Alternate deployment strategies (e.g. using a soluble plug - discussed below) may also prevent damage to the marker during deployment.

The shape memory marker 400 has been shown to be visible under several different imaging modalities. For example, ultrasound imaging of the shape memory marker 400 demonstrated distinct echogenic contrast between the hyperechoic Nitinol edge and hypoechoic hydrogel interior. The resulting effect is a dark interior shape with a bright "outline" when viewed in cross-section. The Nitinol wire configuration is easily visualized as a thin hyperechoic crisscrossed structure with a hypoechoic background when the ultrasound transducer is scanned of the marker. The crimp beads 406 (which may be composed of silver plated brass, for example) on either end of the cage 402 also provide distinct hyperechoic contrast. The fully deployed shape memory biopsy marker 400 is visible under x-ray, which clearly captures the Nitinol wire in the braided shape of the cage 402 as well as the crimp beads 406 on either side of the marker 400. Due to the thin wire diameter, the marker 400 does not mask tissue behind the marker 400 and surrounding media, and as such the marker 400 appears translucent (a.k.a, a "mesh screen" effect). Both the Nitinol and hydrogel components are identifiable using MRI. The Nitinol cage 402 appears as a dark outline around the bright hydrogel core 404 in T2 weighted images. In T1 weighted images, the entire marker 400 appears as a dark region.

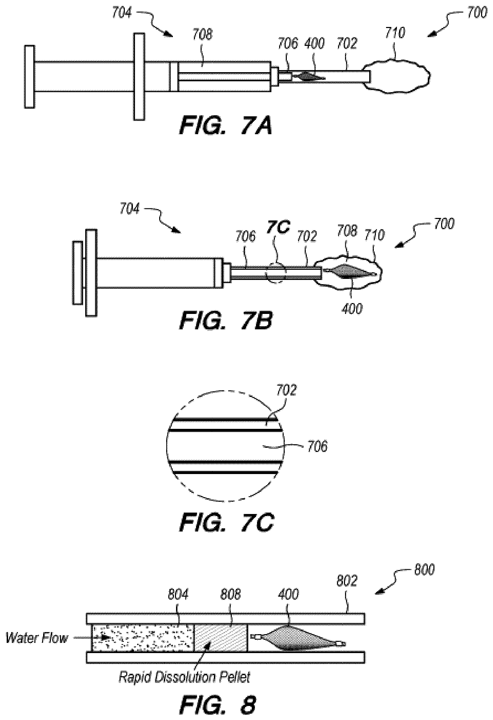

As shown in Figs. 7A and 7B, the biopsy markers described above may be delivered by a delivery device 700 that comprises a cannula 702 with an attached syringe 704 for back flushing with saline 708 and a push rod 706 for deploying the markers. Although Figs. 7A and 7B depict the shape memory marker 400 being deployed, it should be well understood that the delivery device 700 may be used to deliver any of the site markers described above. All of the marker embodiments include a hydrogel component that is designed to provide ultrasound contrast and to assist with migration prevention through swelling and filling a biopsy cavity 710. As such, rapid swelling is preferable for immediate ultrasound imaging capabilities and prevention of migration during delivery cannula 702 retraction. The addition of excess saline 708 upon marker deployment accelerates the swelling process and provides improved ultrasound contrast.

The delivery device 700 allows a one-step deployment that accomplishes delivery of the marker 400 as well as the addition of saline 708 to the biopsy site 710. The biopsy marker 400 may be pre-loaded in the delivery cannula 702, as shown in Fig. 7A. The syringe 704 with the attached push rod 706 may be filled with saline 708 to a desired volume and attached to the cannula 702 containing the marker. Such attachment may be a standard Leuer lock twist fit, or a custom attachment. After positioning the cannula 702 at the biopsy cavity 710, simply pushing the syringe plunger advances the push rod 706, which pushes the biopsy marker 400 into the cavity 610, as shown in Fig. 7B. Simultaneously, saline 708 can be delivered to the biopsy site 710. As shown in greater detail in Fig. 7B, the inner push rod 706 is sized such that the outer diameter is slightly smaller than the inner diameter of the cannula 702, which allows the flow of saline 708 between the push rod surface and the cannula 702.

Having separate cannulas and syringes may allow for sale of the individual components, further minimizing the cost of the marker. The marker could be sold within a disposable cannula, ready for deployment, and medical facilities could have one or more attachable syringe devices on hand.

In another embodiment, shown in Fig. 8, a delivery device 800 comprises a water soluble pellet 808 (e.g., composed of sucrose, PEG, or the like) rather than a push rod. The water soluble pellet 808 is positioned in a cannula 802 between the biopsy marker 400 and saline 804, and is forced forward by fluid pressure during depression of the syringe plunger (not shown). The plunger displaces the biopsy marker 400 into the biopsy cavity as well as the water soluble pellet 808, which will eventually dissolve. Once the water soluble pellet 808 is displaced into the biopsy cavity, the saline solution 804 is free to fill the cavity and contribute to hydration of the biopsy marker 400. An advantage of this embodiment is that the soluble pellet 808 distributes the force of the deployment and reduces possibility for damage to the biopsy marker.

Each of the embodiments described above with reference to Figs. 4A-4B comprises a hydrogel for enhanced ultrasound and MRI contrast and for limiting marker migration. The expansion behavior post deployment serves to center the marker within the biopsy cavity and prevent migration. The hydrogel is castable/moldable which allows for the marker to be manufactured in a variety of shapes. The hydrogel may be loaded with other additives such as radiopacifiers and can potentially be bonded to underlying substrates depending on their surface chemistry.

The shape memory marker 400, while slower at reaching the equilibrium swell state, will immediately absorb water and form a hydrated shell around a dehydrated core. As such, all markers described above should be visible under ultrasound and MRI shortly after deployment, but may require about an hour to reach full hydration.

The hydrogel may be composed of polyacrylamide. Polyacrylamide hydrogels commonly have swell ratios in the range of 100-1000 (wt/wt), and a 500-1000 µm dry bead may swell up to 1-2cm in size depending on the nature of the aqueous solvent (e.g., pH, salt content). The mechanical properties of the polyacrylamide may be adjusted by tuning the crosslink density, resulting in a softer or more firm gel upon full hydration. Polyacrylamide is resistant to biological degradation, so the properties and imaging contrast should be stable for more than fifty-two weeks. The swelling kinetics of polyacrylamide will depend on the surface area to volume ratio of the material. As the surface area to volume ratio increases (which will be generally true for smaller markers), the time required to reach an equilibrium volume will decrease. The material used for the hydrogel may also be a combination of one or more of: poly(2-hydroxyethyl methacrylate) (pHEMA), polyvinylpyrrolidone (PVP), poly(ethylene glycol) diacrylate (PEGDA), poly(vinyl alcohol) (PVA). For example, the shape memory marker 400 may employ a co-polymer of PVP/PEGDA. It should be appreciated that material selection may affect reaction to biological fluids. Certain materials (e.g. polyacrylamide) will be sensitive to the ionic character of the surrounding fluid and will exhibit different swell ratios (and therefore imaging contrast by U/S and MRI) when exposed to blood vs interstitial fluid vs DI water. Selecting a material that is relatively insensitive to ionic character of the surrounding fluid (e.g. as the PVP/PEGDA and PVP/PEGDA/PVA) ensures a more predictable and consistent response during end use.

Once a material or materials are selected, many variables remain which will affect the functional performance of the hydrogel. The functional performance of the hydrogel material includes its ultrasound contrast, MRI contrast, swelling kinetics, swell ratio at equilibrium, mechanical properties (dry and swollen state), biocompatibility, degradability, processability, manufacturability, compatibility within the larger marker construct, and even radiopacity. These performance parameters are related to physical properties of the hydrogel such as the solid content, the crosslink density, and the molecular weight between crosslinks. In turn, these may be tuned by adjusting the hydrogel monomer content, relative ratio of monomers (in the case of a co-polymer), the molecular weight of the starting materials, the initiator content, the curing time/temperature/UV dose, or additives such as plasticizers or porogens. For example, with a PVP hydrogel prepared by direct UV crosslinking, the higher the molecular weight of the starting material, the greater the ultrasound contrast of the hydrogel. This is due to increased molecular weight between crosslinks, which allows for a looser polymer network to form, which allows for greater equilibrium water content.

The majority of commercially available biopsy markers employ a metallic component for radiographic contrast. These may be in the form of metal tubing, wires or coils, which present a limited number of shapes that may be obtained without complex manufacturing, or in the form of micro-machined shapes, which can be expensive to produce on a large scale. Use of a radiopacifier loaded polymer as the radiopaque element in the above-described embodiments may allow for such designs to be injection molded or extruded into a variety of shapes in a more cost-effective manner.

In one embodiment, the radiopaque polymer may be PMMA loaded with barium sulfate, specifically employing a modified formulation of commercially available bone cement, KYPHON HV-R, supplied by Medtronic. Modified formulations may be generated from the starting material by preparing bone cement samples with barium sulfate concentrations ranging from 30% (unmodified base material) to 70% by weight. As the barium sulfate concentration increases, the physical integrity of the final material decreases and the x-ray contrast of the PMMA formulations increases. At 70% wt/wt concentration of barium sulfate the PMMA is qualitatively observed to be brittle in nature. A 60% wt/wt concentration of barium sulfate may be preferable for preparation of radiopaque elements in the gel mesh marker 200 and the swelling bead marker 500. The PMMA marker loaded with 60% barium sulfate may achieve qualitatively similar radiographic contrast at a fraction of the size of a commercially available PEKK marker. This may allow for overall miniaturization of the PMMA marker and deployment through smaller gauge delivery devices.

A secondary benefit of the PMMA as a carrier material for the radiopacifier may be the presence of acrylate groups on the surface of the material. For the hydrogel systems studied here, which are partially formed by crosslinking which occurs at the acrylate end groups of the PEGDA chains, the acrylate groups on the surface of the PMMA radiopaque marker can react with the hydrogel and form covalent crosslinks, resulting in a strong bond between the two materials in the case of the swelling bead embodiment. Such a reaction would not be possible on many other surfaces (e.g., Nitinol, titanium) without complex surface modification.

In another embodiment, the radiopaque element may be a radiopacifier-loaded fiber. The advantage of this form is that for woven or braided constructs, such as the gel mesh, curly fry, or shape memory embodiments, a radiopaque polymer fiber may be incorporated into the larger marker in a patterned form to provide a unique radiographic shape for marker identification. The radiopacifier loaded fiber may be a Dyneema RP dtex 135 TS1000 UHMWPE fiber loaded with 17-23% bismuth trioxide by weight.

All of the markers are identifiable under three imaging modalities - ultrasound, x-ray, and MRI. Under ultrasound, the markers are similar in that the hydrogel component appears as a dark region due to the high water content. The Nitinol cage 402 in the shape memory marker 400 appears as a hyperechoic outline around the hydrogel hypoechoic core 404. The Nitinol cage has a distinct ultrasound contrast (without any posterior acoustic shadowing) making it to be easily identified by either an expert radiologist and also for people with limited knowledge in this area. All markers are located in x-ray images by bright white (x-ray absorbing) regions. The shape memory marker 400 is different from the other markers under x-ray, as the entire marker is viewed in the images. The Nitinol cage 402 appears distinct, with the crimp beads 406 showing as larger bright regions. The small diameter of the Nitinol tubing/wire, the Nitinol tubing/wire orientation, the picks per inch (or porosity of the Nitinol cage) prevent the contrast from masking tissue behind the shape memory marker permanently 400. The other markers only have a radiopaque element in the center or at one end of the marker, but still the contrast is sufficient for localizing the markers.

Under T2 weighted MRI images, the markers appear similar in that the hydrogel component appears as a bright region due to the high signal from the water content. The shape memory marker 400 have dark regions/outlines surrounding the hydrogel due to the Nitinol cage 402. The hydrogel appears bright, while the void space within the marker shows as a dark region. True "voids" may be gradually filled by tissue ingrowth after implantation, and this feature of the ultrasound contrast will gradually decrease in magnitude, ultimately disappearing. Under T1 weighted MRI images, the hydrogel components of the markers appear as dark shapes.

## Claims

1. A shape memory-based biopsy marker (400) comprising:
a superabsorbent hydrogel core (404);
a cage (402) surrounding the hydrogel core (404), wherein the cage (402) comprises a superelastic shape memory material; and
wherein ends of the cage (402) are closed off by
crimp beads (406),
welding, or
knotting,
thereby enclosing the hydrogel component (404) within the cage (402) to provide X-ray identification;
**characterized in that** the cage (402) has a teardrop-like shape.

2. The marker (400) of claim 1, wherein the cage is made of Nitinol.

3. The marker (400) of claim 1 or claim 2, wherein ends of the cage (402) are closed off by crimp beads (406).

4. The marker (400) of claim 3, wherein the crimp beads (406) are metallic, wherein preferably the crimp beads (406) comprise titanium, gold, or silver.

5. The marker (400) of claim 3, wherein the crimp beads (406) are made of a polymer.

6. The marker (400) of claim 2, wherein the cage is constructed using Nitinol having a shape transition temperature approximately the same as body temperature.

7. The marker (400) of any preceding claim, wherein the cage (402) is made of braided Nitinol tubing, preferably wherein the Nitinol tubing is highly compressible through radial compression and highly expandable through axial compression.

8. The marker (400) of claim 7, wherein the Nitinol tubing elastically contracts to its native state when released from either a radial or an axial compressive force.

9. The marker (400) of claim 2, wherein the marker (400) is configured to be compressed to a smaller diameter such that the Nitinol tubing forming the cage (402) are in contact with the hydrogel core (404).

10. The marker (400) of claim 2, wherein the diameter of the cage (402) is approximately 0.5 cm at the maximum point, and wherein the length of the cage (402) is approximately 1.0 cm.

11. The marker (400) of any preceding claim, wherein the hydrogel core (404) is approximately 0.5 cm long and 0.8 mm in diameter in the dehydrated state.

12. The marker (400) of any preceding claim, wherein the hydrogel core (404) is a poly(vinyl pyrrolidone-co-ethylene glycol dimethacrylate) (PVPEGDA) gel prepared with the following formulation: 7.5% vinyl pyrrolidone, 2.5% poly(ethylene glycol) dimethyacrylate (700g/mole), and 1% sodium chloride in water.

13. The marker (400) of any preceding claim, wherein the marker (400) is coated with a thin hydrogel layer, preferably with polyethylene glycol (PEG), ePTFE, silicone, poly(glycerol sebacate) PGS.

## Patentansprüche

1. Formgedächtnisbasierter Biopsiemarker (400), umfassend:
einen superabsorbierenden Hydrogelkern (404);
einen den Hydrogelkern (404) umgebenden Käfig (402), wobei der Käfig (402) ein superelastisches Formgedächtnismaterial umfasst; und
wobei Enden des Käfigs (402) durch
Quetschperlen (406),
Schweißen oder
Verknoten
verschlossen sind, wodurch die Hydrogelkomponente (404) innerhalb des Käfigs (402) zur Bereitstellung einer Röntgenidentifizierung eingeschlossen wird;
**dadurch gekennzeichnet, dass** der Käfig (402) eine tropfenartige Form aufweist.

2. Marker (400) nach Anspruch 1, wobei der Käfig aus Nitinol hergestellt ist.

3. Marker (400) nach Anspruch 1 oder Anspruch 2, wobei Enden des Käfigs (402) durch Quetschperlen (406) verschlossen sind.

4. Marker (400) nach Anspruch 3, wobei die Quetschperlen (406) metallisch sind, wobei die Quetschperlen (406) vorzugsweise Titan, Gold oder Silber umfassen.

5. Marker (400) nach Anspruch 3, wobei die Quetschperlen (406) aus einem Polymer hergestellt sind.

6. Marker (400) nach Anspruch 2, wobei der Käfig unter Verwendung von Nitinol mit einer Formumwandlungstemperatur, die ungefähr der Körpertemperatur entspricht, aufgebaut ist.

7. Marker (400) nach einem der vorhergehenden Ansprüche, wobei der Käfig (402) aus geflochtenem Nitinol-Schlauch hergestellt ist, wobei der Nitinol-Schlauch vorzugsweise durch radiale Kompression in hohem Maße komprimierbar und durch axiale Kompression in hohem Maße expandierbar ist.

8. Marker (400) nach Anspruch 7, wobei sich der Nitinol-Schlauch elastisch in seinen Ausgangszustand zusammenzieht, wenn er von einer radialen oder einer axialen Druckkraft entlastet wird.

9. Marker (400) nach Anspruch 2, wobei der Marker (400) dazu ausgebildet ist, auf einen kleineren Durchmesser komprimiert zu werden, so dass der den Käfig (402) bildende Nitinol-Schlauch in Kontakt mit dem Hydrogelkern (404) steht.

10. Marker (400) nach Anspruch 2, wobei der Durchmesser des Käfigs (402) an der größten Stelle ungefähr 0,5 cm beträgt und wobei die Länge des Käfigs (402) ungefähr 1,0 cm beträgt.

11. Marker (400) nach einem der vorhergehenden Ansprüche, wobei der Hydrogelkern (404) im dehydrierten Zustand ungefähr 0,5 cm lang ist und einen Durchmesser von 0,8 mm aufweist.

12. Marker (400) nach einem der vorhergehenden Ansprüche, wobei der Hydrogelkern (404) ein mit der folgenden Formulierung hergestelltes Poly(vinylpyrrolidon-co-ethylenglykoldimethacrylat)-(PVPEGDA)-Gel ist: 7,5 % Vinylpyrrolidon, 2,5 % Poly(ethylenglykol)dimethacrylat (700 g/Mol) und 1 % Natriumchlorid in Wasser.

13. Marker (400) nach einem der vorhergehenden Ansprüche, wobei der Marker (400) mit einer dünnen Hydrogelschicht beschichtet ist, vorzugsweise mit Polyethylenglykol (PEG), ePTFE, Silikon, Poly(glycerolsebacat) PGS.

## Revendications

1. Marqueur de biopsie (400) basé sur une mémoire de forme comprenant :
un noyau d'hydrogel superabsorbant (404) ;
une cage (402) entourant le noyau d'hydrogel (404), dans lequel la cage (402) comprend un matériau à mémoire de forme superélastique ; et
dans lequel des extrémités de la cage (402) sont fermées par
des perles à écraser (406),
soudage, ou
nouage,
en enfermant ainsi le composant hydrogel (404) à l'intérieur de la cage (402) pour permettre une identification par rayons X ;
**caractérisé en ce que** la cage (402) a une forme de type larme.

2. Marqueur (400) selon la revendication 1, dans lequel la cage est constituée de Nitinol.

3. Marqueur (400) selon la revendication 1 ou la revendication 2, dans lequel des extrémités de la cage (402) sont fermées par des perles à écraser (406).

4. Marqueur (400) selon la revendication 3, dans lequel les perles à écraser (406) sont métalliques, dans lequel de préférence les perles à écraser (406) comprennent du titane, de l'or ou de l'argent.

5. Marqueur (400) selon la revendication 3, dans lequel les perles à écraser (406) sont constituées d'un polymère.

6. Marqueur (400) selon la revendication 2, dans lequel la cage est construite en utilisant du Nitinol ayant une température de transition de forme approximativement identique à la température corporelle.

7. Marqueur (400) selon l'une quelconque des revendications précédentes, dans lequel la cage (402) est constituée d'un tubage en Nitinol tressé, de préférence dans lequel le tubage en Nitinol est fortement compressible par compression radiale et fortement extensible par compression axiale.

8. Marqueur (400) selon la revendication 7, dans lequel le tubage en Nitinol se contracte élastiquement jusqu'à son état natif lorsqu'il est libéré d'une force de compression radiale ou axiale.

9. Marqueur (400) selon la revendication 2, dans lequel le marqueur (400) est configuré pour être comprimé jusqu'à un diamètre plus petit de sorte que le tubage en Nitinol formant la cage (402) est en contact avec le noyau d'hydrogel (404).

10. Marqueur (400) selon la revendication 2, dans lequel le diamètre de la cage (402) est d'approximativement 0,5 cm au point maximal, et dans lequel la longueur de la cage (402) est d'approximativement 1,0 cm.

11. Marqueur (400) selon l'une quelconque des revendications précédentes, dans lequel le noyau d'hydrogel (404) a une longueur d'approximativement 0,5 cm et un diamètre de 0,8 mm dans l'état déshydraté.

12. Marqueur (400) selon l'une quelconque des revendications précédentes, dans lequel le noyau d'hydrogel (404) est un gel de poly(vinylpyrrolidone-co-éthylène glycol diméthacrylate) (PVPEGDA) préparé avec la formulation suivante : vinylpyrrolidone à 7,5 %, poly(éthylène glycol) diméthacrylate à 2,5 % (700 g/mole) et chlorure de sodium à 1 % dans de l'eau.

13. Marqueur (400) selon l'une quelconque des revendications précédentes, dans lequel le marqueur (400) est enrobé d'une fine couche d'hydrogel, de préférence de polyéthylène glycol (PEG), d'ePTFE, de silicone, de poly(sébacate de glycérol) PGS.
